# EUROPÄISCHE PATENTSCHRIFT

(11) **EP 3 093 074 B1**
(45) Veröffentlichungstag und Bekanntmachung des Hinweises auf die Patenterteilung: **04.12.2019**
(21) Anmeldenummer: 15167614.5
(22) Anmeldetag: 13.05.2015
(51) Int. Cl.: B05B 11/00, A61M 15/08, B65D 47/24, A61M 15/00, B65D 50/04

(54) **AUSTRAGKOPF FÜR EINEN SPENDER ZUM AUSTRAG EINER FLÜSSIGKEIT SOWIE SPENDER MIT EINEM SOLCHEN AUSTRAGKOPF UND BEFESTIGUNGSABSCHNITT FÜR EINEN SOLCHEN AUSTRAGKOPF**
APPLICATOR HEAD FOR A DISPENSER FOR DISCHARGING A LIQUID, AS WELL AS DISPENSER WITH SUCH A DISCHARGE HEAD AND ATTACHMENT SECTION FOR SUCH AN APPLICATOR HEAD
TÊTE DISTRIBUTRICE POUR UN DISTRIBUTEUR POUR LA SORTIE D'UN LIQUIDE ET DISTRIBUTEUR DOTÉ D'UNE TELLE TÊTE DISTRIBUTRICE ET SECTION DE FIXATION POUR UNE TELLE TÊTE DISTRIBUTRICE

(43) Veröffentlichungstag der Anmeldung: 16.11.2016
(73) Patentinhaber: Aptar Radolfzell GmbH, 78315 Radolfzell (DE)
(72) Erfinder: Ritsche, Stefan, 78253 Eigeltingen (DE)
(74) Vertreter: Patentanwaltskanzlei Cartagena

(56) Entgegenhaltungen:
- EP-A1- 0 653 359
- WO-A1-2008/001406
- CN-A- 104 340 501
- DE-B3-102013 202 933

## Beschreibung

### ANWENDUNGSGEBIET UND STAND DER TECHNIK

Die Erfindung betrifft einen Austragkopf für einen Spender zum Austrag einer Flüssigkeit, insbesondere zum Austrag einer pharmazeutischen oder kosmetischen Flüssigkeit, nach dem Oberbegriff von Anspruch 1. Die Erfindung betrifft weiterhin einen Spender nach dem Oberbegriff von Anspruch 11.

Gattungsgemäße Austragköpfe sowie Spender mit solchen Austragköpfen sind allgemein bekannt. Sie finden zum Austrag pharmazeutischer Flüssigkeiten Verwendung, wie beispielsweise zum Austrag von Medikamenten oder salzwasserhaltigen Lösungen. Durch Niederdrücken des bei gattungsgemäßen Spendern vorgesehenen Applikators werden eine Flüssigkeitsförderung zur Austragöffnung und ein Flüssigkeitsaustrag in die Umgebung verursacht.

Da gattungsgemäße Spender vor allem für den Austrag von pharmazeutischen Flüssigkeiten, wie z.B. imidazolinhaltigen Substanzen verwendet werden, besteht der Bedarf, solche Spender mit einer Kindersicherung zu versehen. Diese verhindert, dass kleine Kinder einen Flüssigkeitsaustrag verursachen können und so an das möglicherweise gefährliche Medikament gelangen.

Kindersicherungen an Flüssigkeitsspendern sind aus dem Stand der Technik ebenfalls vielfach bekannt. Es handelt sich zumeist um Kindersicherungen, die ein Abnehmen der Kappe oder ein Betätigen des Spenders erschweren. Üblicherweise sind solche Spender mit Kindersicherungen bereits im Rahmen ihrer Entwicklung entsprechend ausgestaltet worden.

Es gibt jedoch auch eine Vielzahl bekannter Spendermodelle, die ohne eine solche Kindersicherung konzipiert wurden. Solche Spender sind üblicherweise im Falle von insbesondere regulatorisch vorgegebenen Kindersicherungen nicht weiterverwendbar, so dass häufig eine Neukonstruktion mit einer Vielzahl neuer Bauteile erforderlich wird.

Aus der DE 102013202933 B3 und der WO 2008/001406 A1 sind Spender bekannt, die jeweils ein Zwischenglied vorsehen, welches zwischen einer Kappe und der Basis eines Austragkopfes angeordnet ist. Im Falle der WO 2008/001406 A1 handelt es sich um ein mehrteiliges Zwischenglied, welches eine Kindersicherung bildet, indem es das Abnehmen der Kappe erschwert.

Aus Dokument DE 10 2013 202 933 B3 sind Spendergestaltungen mit zweiteiligen Kappen bekannt, wobei die Elemente der Kappe durch Kraftbeaufschlagung formschlüssig verbindbar sind, um im kraftbeaufschlagten Zustand gemeinsam zur Freigabe einer Austragöffnung abnehmbar zu sein.

Aus der CN 104340501 A ist ein Austragkopf bekannt, bei dem Kopfstück eines Spenders zweistückig ausgebildet ist und neben einem Applikatorteil mit Austragöffnung ein Fingerauflagenteil aufweist, an dem Rastelemente für eine Kappe angebracht sind. Ein Außengewinde zur Befestigung der Kappe ist am Applikatorteil vorgesehen.

### AUFGABE UND LÖSUNG

Aufgabe der Erfindung ist es, eine Möglichkeit zur Verfügung zu stellen, Spender sowie Austragköpfe solcher Spender, die nicht über eine Kindersicherung verfügen, möglichst einfach um eine solche Kindersicherung zu ergänzen, so dass eine Vielzahl von Bauteilen des Spenders unverändert bleiben kann.

Die der Erfindung zugrunde liegende Aufgabe wird durch einen Austragkopf nach Anspruch 1 gelöst.

Der Austragkopf umfasst eine Basis und einen beweglich an der Basis angebrachten Applikator. Die Basis ist zur Montage an einem Flüssigkeitsspeicher oder zur festen Integration an einen Flüssigkeitsspeicher ausgebildet. Der Applikator umfasst eine Austragöffnung, durch die Flüssigkeit in eine Umgebung abgegeben werden kann. Der Austragkopf umfasst eine Pumpeinrichtung oder eine Ventileinrichtung, die in einem Flüssigkeitskanal angeordnet ist, der den Flüssigkeitsspeicher mit der Austragöffnung verbindet, wobei die Pumpeinrichtung durch Verlagerung des Applikators gegenüber der Basis betätigbar ist oder wobei die Ventileinrichtung durch Verlagerung des Applikators gegenüber der Basis geöffnet und geschlossen werden kann.

Der Austragkopf umfasst eine kindergesicherte Kappeneinheit. Die Kappeneinheit umfasst eine Kappe zur Überdeckung der Austragöffnung. Die Kappeneinheit umfasst weiter einen separaten Befestigungsabschnitt, der über eine Befestigungseinrichtung einerseits fest an der Basis oder dem Applikator angebracht ist und der andererseits zur lösbaren Befestigung der Kappe ausgebildet ist.

Der erfindungsgemäße Austragkopf verfügt demnach über eine Kappe, die Kindern den Zugang zur Austragöffnung erschweren soll. Diese Kappe ist am Applikator gesichert, wobei diese Sicherung mittelbar über den genannten Befestigungsabschnitt erfolgt. Der Applikator kann somit unverändert so ausgestaltet sein, wie ein nicht zur Kindersicherung vorgesehener Applikator ausgestaltet ist. Erst durch die Anbringung des Befestigungsabschnitts der Kappeneinheit wird ein Gegenstück zur Verfügung gestellt, welches zusammenwirkend mit der Kappe deren kindergesicherte Festlegung am Applikator ermöglicht.

Dies bedeutet, dass der Austragkopf bereits ohne erfindungsgemäß vorgesehene Kappeneinheit hinsichtlich seiner Hauptfunktion, des Flüssigkeitsaustrags, voll funktionstauglich ist. Die Kappeneinheit ergänzt lediglich die Kindersicherungsfunktionalität und kann weggelassen werden, sofern eine solche Kindersicherungsfunktionalität nicht erforderlich ist.

Der separate Befestigungsabschnitt ist über die Befestigungseinrichtung mit dem Applikator verbunden. Diese Befestigung soll bestimmungsgemäß ausreichend stabil sein, dass ein Kind sie nicht lösen kann. Begünstigt wird die Kindersicherung dadurch, dass die grundsätzliche Zweiteiligkeit des Applikators einerseits und des Befestigungsabschnitts andererseits für das Kind nicht ersichtlich sein muss. Es wird daher eher versuchen, die Kappe vom Befestigungsabschnitt zu lösen, als die gesamte Kappeneinheit vom Applikator zu lösen.

Die Kappe kann an ihrer Innenseite über eine Dichtfläche verfügen, mittels derer im aufgesetzten Zustand die Austragöffnung unmittelbar verschlossen wird. Hierdurch kann besonders sicher verhindert werden, dass Kinder an die Flüssigkeit gelangen, da diese selbst bei Betätigung mit aufgesetzter Kappe nicht bis in den Kappeninnenraum stromabwärts der Austragöffnung gelangen.

Die Kappe kann auch einen Blockierabschnitt aufweisen, der zusammen mit einem weiteren Blockierabschnitt an der Basis im aufgesetzten Zustand der Kappe ein Niederdrücken des Applikators gegenüber der Basis verhindert. Bei einer solchen Gestaltung wird somit bereits verhindert, dass Flüssigkeit in Richtung der Austragöffnung gefördert wird. Auch so kann wirksam verhindert werden, dass Flüssigkeit in den Kappeninnenraum stromabwärts der Austragöffnung gelangen kann.

Der Befestigungsabschnitt der Kappeneinheit kann als zumindest abschnittsweise hülsenförmiger und beidseitig offener Körper gestaltet sein, der den Applikator umgibt.

Die Hülsenform des Befestigungsabschnittes ist vorgesehen, um diesen insbesondere von der Austragöffnung aus auf den Applikator aufschieben zu können, wobei dies vorzugsweise schon bei der Herstellung erfolgt. Wenngleich die Hülse auch geschlitzt ausgebildet sein könnte, ist sie vorzugsweise umlaufend geschlossen. Dies ermöglicht es, sie besonders fest am Applikator befestigen zu können und somit eine Trennung des Befestigungsabschnitts vom Applikator zu verhindern. Weiterhin gestattet es die Hülsenform, den Charakter des Befestigungsabschnitts als separatem Bauteil zu verstecken.

Der Befestigungsabschnitt und die Kappe der Kappeneinheit sind mit einer Gewindeeinrichtung oder einer Bajonetteinrichtung zum Befestigen der Kappe am Befestigungsabschnitt versehen.

Die Gewindeeinrichtung bzw. die Bajonetteinrichtung erzwingt, dass die Trennung der Kappe vom Befestigungsabschnitt mit einer kombinierten rotativen und translativen Bewegung einhergehen muss. Für Kleinkinder stellt bereits dies einen besseren Schutz dar als eine einfach abziehbare Kappe. Vorzugsweise ist eine solche Gewindeeinrichtung oder Bajonetteinrichtung jedoch nur ein Teil der Kindersicherung, die darüber hinaus weitere Hürden für Kinder bereithält. Unter einer Gewindeeinrichtung im Zusammenhang mit der Erfindung wird jegliche durch gegenüber der Haupterstreckungsrichtung des Applikators schräg gestellte Führungsfläche verstanden, die eine gleichzeitige rotative und lineare Relativbewegung der Kappe gegenüber dem Befestigungsabschnitt zum Zwecke des Trennens erforderlich macht.

Der Befestigungsabschnitt und die Kappe der Kappeneinheit können über eine Drehsicherungseinrichtung verfügen, durch die die Kappe und der Befestigungsabschnitt in einer definierten Drehstellung formschlüssig gegen Verdrehen gesichert werden können. Dabei können der Befestigungsabschnitt oder die Kappe abschnittsweise derart verformbar sein, dass hierdurch die durch die Drehsicherungseinrichtung herstellbare Drehsicherung lösbar ist.

Eine solche Drehsicherungseinrichtung verhindert nach Aufsetzen der Kappe auf den Befestigungsabschnitt, dass eine ungehinderte rotative Relativbewegung möglich wird, wie sie zum Trennen beispielsweise eines Gewindes erforderlich ist. Es bedarf somit zunächst des Lösens der Drehsicherung, um anschließend durch eine zumindest auch rotative Relativbewegung die Kappe vom Befestigungsabschnitt trennen zu können. Besonders bevorzugt ist es, wenn durch eine radiale Verformung der Kappe oder gegebenenfalls auch des Befestigungsabschnitts die Drehsicherung gelöst wird.

Vorzugsweise sind radiale Einbuchten oder Ausstülpungen am Befestigungsabschnitt vorgesehen, die im drehgesicherten Zustand korrespondierende Ausstülpungen oder Einbuchtungen an der Kappe aufnehmen. Da ausgehend von aufgeschraubten Zustand nur eine Drehbewegung in Abschraubrichtung erzielt werden muss, können statt der Einbuchtungen und Ausstülpungen auch nur in eine Drehrichtung beschränkende Anschlagflächen dienen.

Das Ausrücken zum Zwecke anschließenden Drehens wird vorzugsweise dadurch erreicht, dass die Kappe um 90° gegenüber diesen Ausbuchtungen und Einstülpungen oder gegenüber aneinander anliegenden Anschlägen versetzt zusammengedrückt wird, so dass das genannte Ausrücken stattfindet.

Die Kappe kann als Doppelkappe mit einer Innenkappe und einer Außenkappe gestaltet sein. Dabei können die Innenkappe und die Außenkappe über zusammenwirkende Kopplungsmittel verfügen, durch die durch Kraftbeaufschlagung der Außenkappe eine temporäre Drehsicherung zwischen der Außenkappe und der Innenkappe herstellbar ist.

Bei einer solchen Gestaltung sind die Innenkappe und die Außenkappe grundsätzlich gegeneinander drehbar, jedoch zumindest bestimmungsgemäß nicht voneinander trennbar. Durch eine radiale oder axiale Kraftbeaufschlagung der Außenkappe wird diese in kraftschlüssigen oder insbesondere formschlüssigen Eingriff mit der Innenkappe verbracht, so dass ein gemeinsames Drehen möglich wird. Dieses Konzept einer Kindersicherung ist für sich genommen bekannt und hat sich als zweckmäßig herausgestellt. Der Befestigungsabschnitt kann bei einer solchen Gestaltung vergleichsweise einfach ausgestaltet sein, weil er jenseits von Gewindegängen keine weiteren Mittel braucht, um mit der genannten Doppelkappe verbindbar zu sein.

Die Befestigungseinrichtung kann eine Rastverbindungseinrichtung umfassen.

Eine solche Rastverbindungseinrichtung stellt einen sehr einfachen Weg dar, um den Befestigungsabschnitt am Applikator festzulegen. Auch ist die Montage hier sehr einfach, da der gleiche Vorgang, durch den der vorzugsweise hülsenförmige Befestigungsabschnitt auf den Applikator aufgeschoben wird, auch ein Fügen dieser Rasteinrichtung gegen Ende der Relativbewegung mit sich bringt.

Die Befestigungseinrichtung kann als drehfeste Befestigungseinrichtung ausgebildet sein, durch die der Befestigungsabschnitt einerseits und der Applikator andererseits drehfest miteinander verbunden sind.

Durch die Drehfestverbindung des Befestigungsabschnitts mit dem Applikator wird erreicht, dass eine rotative Momentenbeaufschlagung zum autorisierten Abnehmen der Kappe kein Ergreifen des Befestigungsabschnitts einerseits und der Kappe andererseits erforderlich macht. Stattdessen kann der Applikator oder möglicherweise auch die Basis oder der Flüssigkeitsspeicher einerseits und die Kappe andererseits zum Abnehmen ergriffen werden.

Es ist jedoch auch eine andere Gestaltung denkbar, bei der der Befestigungsabschnitt bestimmungsgemäß drehbar am Applikator angebracht ist, damit ein Lösen der Kappe gerade dort eine Momenteneinkopplung erforderlich macht. Dies kann je nach Ausgestaltung des Befestigungsabschnitts eine schwierige Hürde für Kinder darstellen.

Die Befestigungseinrichtung kann zur Kopplung des Befestigungsabschnitts am Applikator ausgebildet sein, wobei die Befestigungseinrichtung eine radial nach außen offene Kopplungsnut umfassen kann, in den ein innenseitig am Befestigungsabschnitt vorgesehener Kopplungssteg eingreift.

Eine Gestaltung, bei der eine nach außen offene Nut am Applikator zur Befestigung des Befestigungsabschnittes vorgesehen ist, hat sich als sehr vorteilhaft herausgestellt. Dabei müssen die Kopplungsnut und der Kopplungssteg nicht zwingend umlaufend sein. Sofern eine ausreichende Verformbarkeit im Zuge der Montage gegeben ist, kann jedoch eine umlaufende Gestaltung vorteilhaft sein. Der Vorteil einer solchen Gestaltung mit Kopplungsnut und Kopplungssteg ist insbesondere gegeben, wenn eine solche Kopplungsnut zur Befestigung einer Fingerauflage ohnehin am Applikator vorgesehen ist. Diese kann dann in der kindergesicherten Variante des Austragkopfes für den Befestigungsabschnitt genutzt werden, der seinerseits eine Fingerauflage zur Verfügung stellt.

Der Befestigungsabschnitt der Kappeneinheit kann über eine Grifffläche verfügen, die zur Erhöhung der Griffigkeit mit Griffrippen oder anderweitigen Grifferhebungen versehen ist.

Sofern vorgesehen ist, dass zum autorisierten Lösen der Kappe ein Ergreifen des Befestigungsabschnitts erforderlich ist, ist eine solche Grifffläche von Vorteil. Dies gilt insbesondere für Austragköpfe, die in einer nicht kindergesicherten Variante keine geeigneten Flächen zur Verfügung stellen, um ein zum Lösen der Kappe erforderliches Drehmoment einzukoppeln.

Der Befestigungsabschnitt der Kappeneinheit kann dabei Fingerauflage umfassen, die vorzugsweise einen Außenrand aufweist, dessen Formgebung von der Kreisform abweicht.

Die Ausgestaltung eines Befestigungsabschnitts mit Fingerauflage bietet sich insbesondere dann an, wenn die bei einem nicht kindergesicherten Spender vorgesehene Fingerauflage bestimmungsgemäß weggelassen wird, um Platz für den Befestigungsabschnitt zu schaffen. Durch einen Außenrand, der nicht kreisförmig ist, wird ein Ergreifen der Fingerauflage zum Zwecke des Lösens der Kappe für den Benutzer erleichtert.

Der Applikator kann als Nasalapplikator ausgebildet sein und über eine Nasenolive verfügen, an deren distalem Ende die Austragöffnung vorgesehen ist.

Austragköpfe mit Nasalapplikatoren können insbesondere zur Verwendung von nasalapplizierten Schmerzmitteln oder imidazolinhaltige oder vaskonstrikutiv wirkende Substanzen Verwendung finden, hinsichtlich derer eine Kindersicherung besonders wichtig ist. Bei solchen Austragköpfen ist der Applikator abschnittsweise schlank ausgebildet, um ein Einführen in das Nasenloch des Patienten zu gestatten. Eine solche schlanke Ausführung steht jedoch der Verwendung eines hülsenförmigen Befestigungsabschnitts nicht entgegen. Dieser kann ausreichend dünnwandig sein, so dass er dennoch die schlanke Gestalt zulässt.

Der Applikator kann als Applikator zur Tropfenabgabe ausgebildet sein und über eine die Austragöffnung umgebende Tropfenbildungsfläche verfügen, an der ausgetragenen Flüssigkeit anhaftet, bis sie sich in Tropfenform hiervor löst.

Ein solcher Applikator verfügt an seiner Außenseite die Austragöffnung umgebend über einen vorzugsweise flachen Bereich, der von einer Abrisskante umgeben wird. Hier sammelt sich in einer Überkopflage des Applikators die Flüssigkeit, bis die Flüssigkeitsmenge ausreichend groß ist, um sich von der Tropfenbildungsfläche zu lösen.

Der Applikator kann eine sich in Richtung auf die Austragöffnung zu verjüngende außenseitige Formgebung aufweisen, und der Befestigungsabschnitt kann eine zur dieser Formgebung des Applikators korrespondierende innenseitige sich verjüngende Formgebung aufweisen.

Insbesondere zur Schaffung eines schlanken Applikators ist ein solches bündiges Anliegen der Innenseite des Befestigungsabschnitts am innen liegenden Applikator zweckmäßig.

An der Fingerauflage kann ein umlaufender Hülsenabschnitt vorgesehen sein, der die Basis umgibt.

Ein solcher Hülsenabschnitt, der vorzugsweise umlaufend ausgebildet ist, kann sich von der Fingerauflage als Teil des Befestigungsabschnitts in Richtung der Basis erstrecken und somit verhindern, dass ein Kind beim Versuch, Flüssigkeit auszutragen, sich einen Finger zwischen Basis und Applikator einklemmt.

Die der Erfindung zugrunde liegende Aufgabe wird auch durch einen Spender nach Anspruch 10 gelöst.

Der Spender umfasst einen Flüssigkeitsspeicher. Der Spender umfasst einen Austragkopf mit einer Austragöffnung, durch die Flüssigkeit in eine Umgebung abgebbar ist.

Dieser Austragkopf ist nach vorbeschriebener Art ausgebildet.

Der Austragkopf und der Flüssigkeitsspeicher eines solchen Spenders können als separate Einheiten ausgebildet sein, die beispielsweise durch ein Gewinde, verzahnte Gehäuse, formschlüssig wirkende Schnappverbindungen oder über Krimpverbindungen verbunden sind. Ebenso sind jedoch Ausgestaltungen denkbar, bei denen die Basis des Austragkopfes fest mit dem Flüssigkeitsspeicher verbunden ist oder fest mit einem Gehäuse verbunden ist, innerhalb dessen der Flüssigkeitsspeicher vorgesehen ist. Hierdurch kann für ein Kind zusätzlich erschwert werden, durch ein Trennen des Austragkopfes und des Flüssigkeitsspeichers an die Flüssigkeit zu gelangen.

Bei der im Spender enthaltenen Flüssigkeit handelt es sich vorzugsweise um ein pharmazeutisches oder kosmetisches Medium. Insbesondere handelt es sich um Schmerzmittel oder anderweitige pharmazeutische Flüssigkeiten, die für Kinder schädlich sind.

Der Flüssigkeitsspeicher kann ein Volumen von maximal 1000ml aufweisen, vorzugsweise von maximal 100ml, insbesondere vorzugsweise von maximal 50ml.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1a bis 1d zeigen exemplarisch eine bekannte Ausgestaltung eines Spenders zum Austrag von Flüssigkeit in geschnittener und perspektivischer Darstellung sowie mit und ohne aufgesetzte Kappe.
Fig. 2a bis 2f zeigen ein erstes Ausführungsbeispiel der Erfindung.

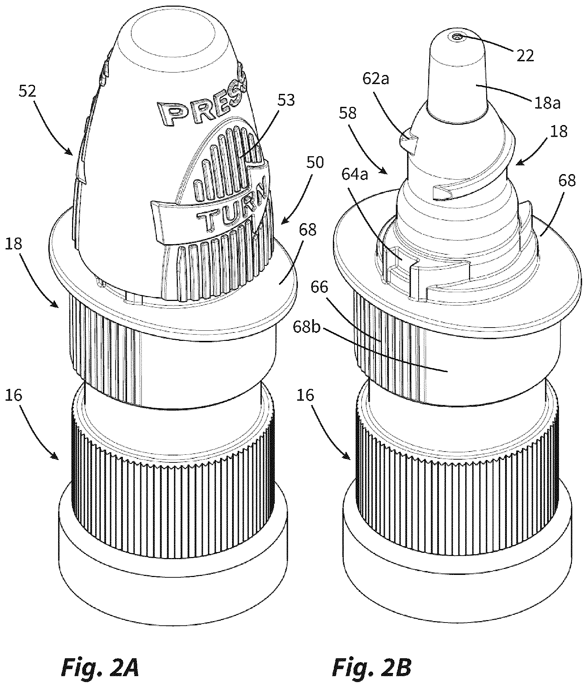

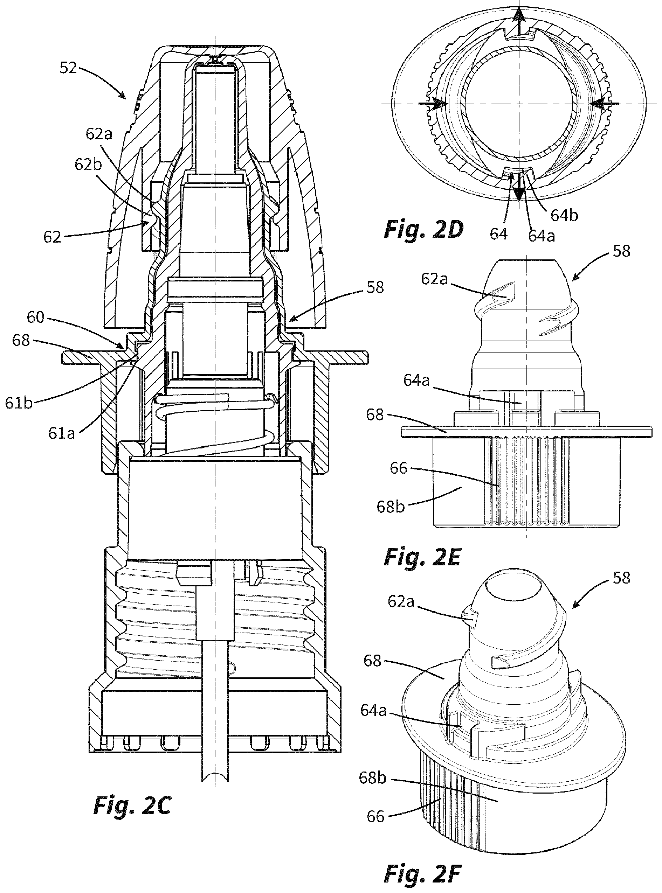

Fig. 2a und 2b zeigen den Austragkopf gemäß diesem ersten Ausführungsbeispiel in perspektivischer Darstellung mit und ohne aufgesetzte Kappe.
Fig. 2c zeigt den Austragkopf gemäß diesem ersten Ausführungsbeispiel in geschnittener Darstellung.
Fig. 2d bis 2f zeigen den Befestigungsabschnitt einer Kappeneinheit des Austragkopfes gemäß diesem ersten Ausführungsbeispiel von oben und von der Seite sowie in perspektivischer Darstellung.

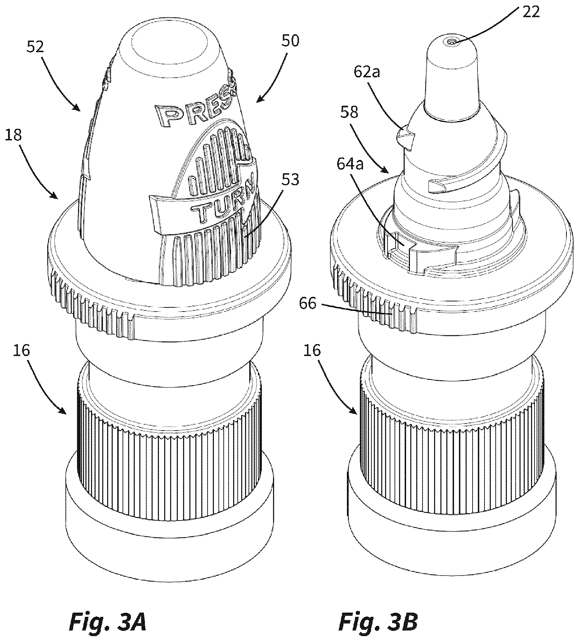

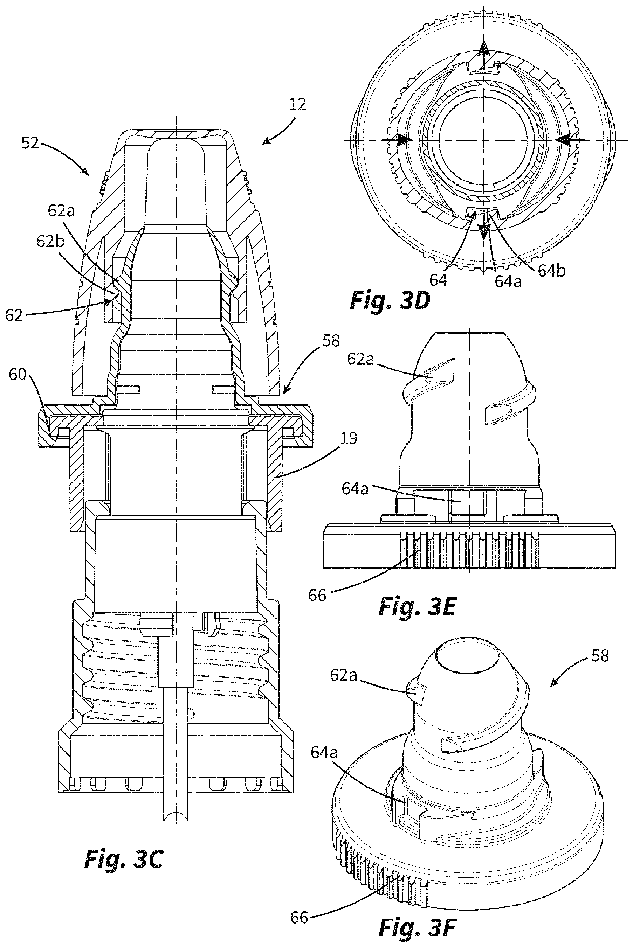

Fig. 3a bis 3f zeigen ein zweites Ausführungsbeispiel der Erfindung.
Fig. 3a und 3b zeigen den Austragkopf gemäß diesem zweiten Ausführungsbeispiel in perspektivischer Darstellung mit und ohne aufgesetzte Kappe.
Fig. 3c zeigt den Austragkopf gemäß diesem zweiten Ausführungsbeispiel in geschnittener Darstellung.
Fig. 3d bis 3f zeigen den Befestigungsabschnitt einer Kappeneinheit des Austragkopfes gemäß diesem zweiten Ausführungsbeispiel von oben und von der Seite sowie in perspektivischer Darstellung.

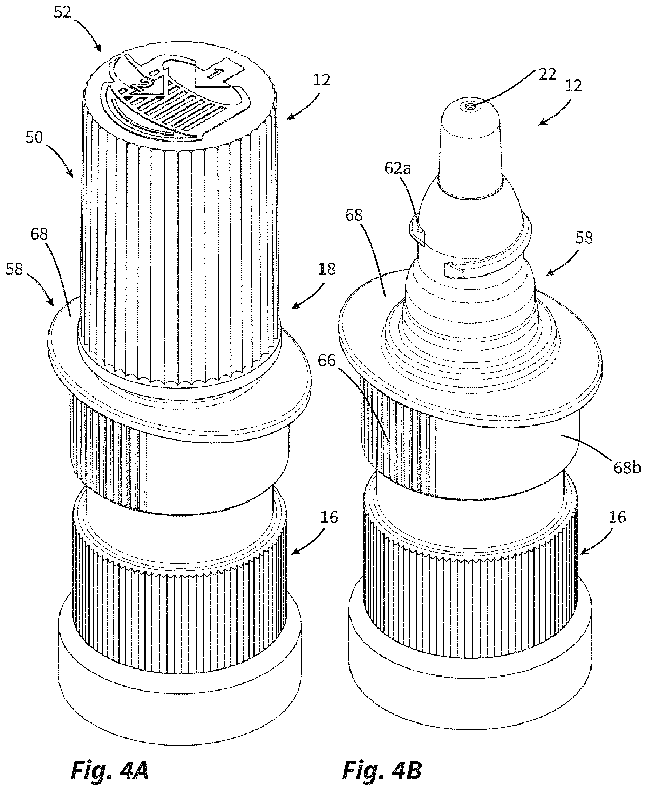

Fig. 4a bis 4e zeigen ein drittes Ausführungsbeispiel der Erfindung.
Fig. 4a und 4b zeigen den Austragkopf gemäß diesem dritten Ausführungsbeispiel in perspektivischer Darstellung mit und ohne aufgesetzte Kappe.

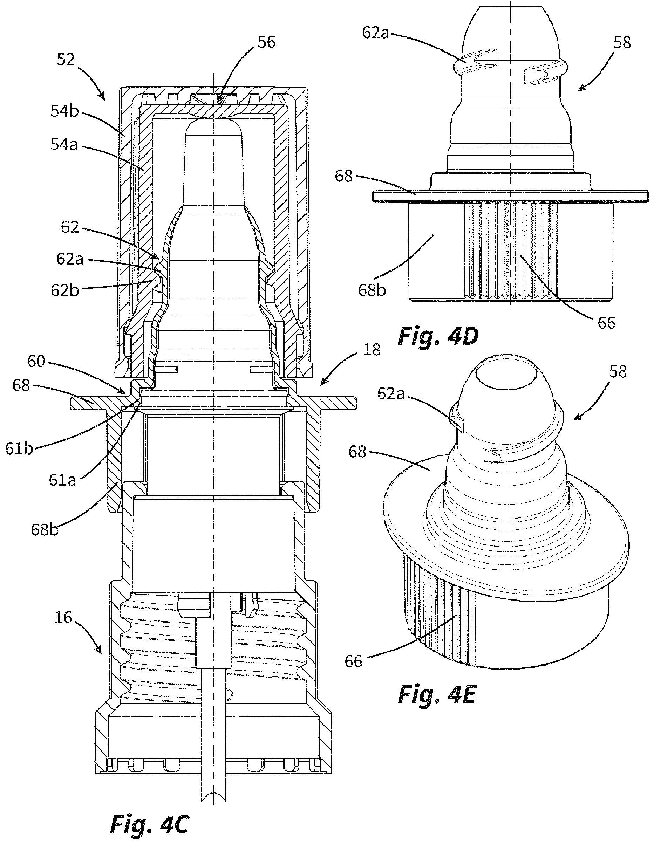

Fig. 4c zeigt den Austragkopf gemäß diesem dritten Ausführungsbeispiel in geschnittener Darstellung.
Fig. 4d und 4e zeigen den Befestigungsabschnitt einer Kappeneinheit des Austragkopfes gemäß diesem dritten Ausführungsbeispiel von der Seite sowie in perspektivischer Darstellung.

### DETAILLIERTE BESCHREIBUNG DER AUSFÜHRUNGSBEISPIELE

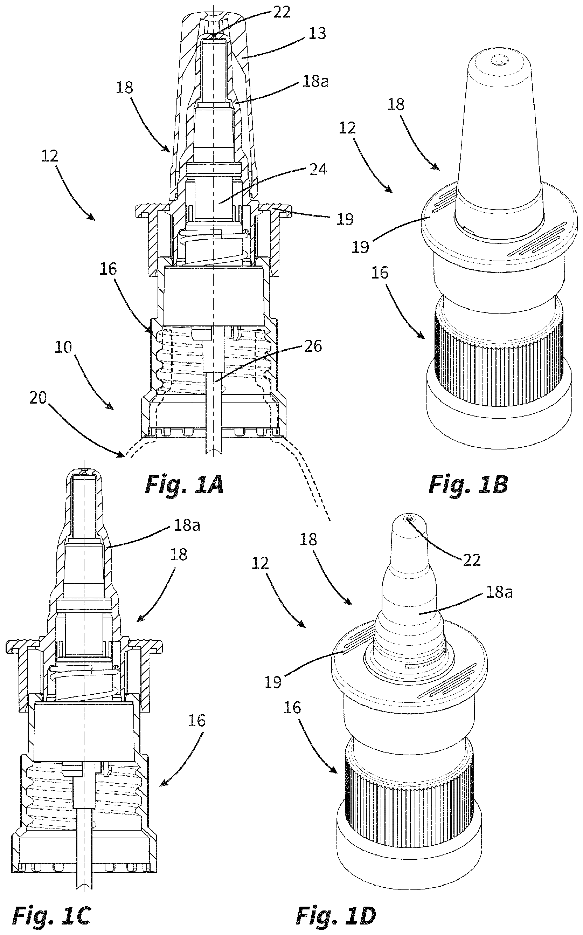

Die Fig. 1A bis 1D zeigen einen Austragkopf 12 eines Flüssigkeitsspenders 10, dessen Flüssigkeitsspeicher in Fig. 1A zum besseren Verständnis gestrichelt dargestellt ist, in den weiten Figuren jedoch zum Zwecke der Vereinfachung ausgeblendet ist.

Ein solcher gattungsgemäßer Austragkopf 12 weist keine Kindersicherung auf. Die Kappe 13 dieses Spenders 10 ist auf einen als Nasalapplikator ausgebildeten Applikator 18 des Austragkopfes 12 lediglich aufgesteckt und kraftschlüssig verrastet. Zwischen einer Basis 16 und dem Applikator 18 des Austragkopfes 12 ist eine Pumpeinrichtung 24 vorgesehen, mittels derer Flüssigkeit aus dem Flüssigkeitsspeicher 20 über einen Flüssigkeitskanal 26 bis zu einer Austragöffnung 22 gefördert werden kann, indem der Applikator 18 niedergedrückt wird.

Statt der Pumpeinrichtung 24 könnte auch eine Ventileinrichtung vorgesehen sein. In einem solchen Falle wäre vorgesehen, dass die Flüssigkeit im Flüssigkeitsspeicher 20 bereits vor Beginn des Austragvorgangs unter Druck steht und im Zuge des Öffnens der Ventileinrichtung in Richtung der Austragöffnung 22 strömt.

Ein Spender wie der in den Fig. 1A bis 1D dargestellte, stellt ein am Markt etabliertes Produkt dar. Es stehen demnach gut funktionierende Werkzeuge zur Herstellung des Spenders zur Verfügung. Um einen solchen Spender mit einer Kindersicherung auszustatten, bietet die Erfindung die Möglichkeit, unter Beibehaltung aller oder fast aller Bauteile des Spenders gemäß der Fig. 1A bis 1D diese Kindersicherung zusätzlich vorzusehen. Neben der Tatsache, dass hierdurch Kosten für neue Werkzeuge und die Etablierung eines vollständig neuen Produktionsprozesses gespart werden, liegt der Vorteil auch darin, dass für mehrere Spendertypen - kindergesicherte und nicht kindergesicherte - die Zahl der Gleichteile hoch ist.

Die Fig. 2A bis 2F zeigen eine erste Variante einer solchen am Spender der Fig. 1A bis 1D vorgesehenen Kindersicherung. Bei dieser Variante finden die Kappe 13 und eine Fingerauflage 19 des Spenders der Fig. 1A bis 1D keine Verwendung mehr. Alle anderen Bauteile bleiben unverändert vorhanden. Bezug nehmend insbesondere auf Fig. 2C verfügt diese erste Ausführungsform eines erfindungsgemäßen Spenders über einen hülsenförmigen Befestigungsabschnitt 58, der gemeinsam mit einer besonders ausgestalteten Kappe 52 eine erfindungsgemäße Kappeneinheit 50 bildet. Der Befestigungsabschnitt 58 ist als beidseitig offener Hohlkörper ausgestaltet, dessen Innenseite im Bereich einer Nasenolive 18a des Applikators 18 an die Formgebung dieser Nasenolive 18a angepasst ist. An der Innenseite des Befestigungsabschnitts 58 ist ein Kopplungssteg 61b vorgesehen, der in eine applikatorseitige Kopplungsnut 61a eingreift und so eine Rastverbindung schafft. Die genannte Kopplungsnut 61a ist jene Nut, in der beim nicht kindergesicherten Spender der Fig. 1A bis 1D die Fingerauflage 19 befestigt wurde. Die Fingerauflage 19 selbst ist durch eine am Befestigungsabschnitt 58 vorgesehene Fingerauflage 68 ersetzt. Diese verfügt ebenso wie die Fingerauflage 19 über einen Hülsenabschnitt 68b, der sich in Richtung der Basis 16 erstreckt und diese bereits im ungedrückten Zustand partiell umgibt, so dass das Einklemmen eines Fingers wirksam verhindert wird.

An der Außenseite des Befestigungsabschnitts 58 weist dieser eine besondere Formgebung auf, um eine kindergesicherte Festlegung der Kappe 52 zu gestatten. Diese Formgebung umfasst ein Außengewinde 62a, welches Teil einer Gewindeeinrichtung 62 ist, sowie zwei Ausnehmungen 64a, die Teil einer Drehsicherungseinrichtung 64 sind. Korrespondierend hierzu sind an der Innenseite der Kappe 52 ein Innengewinde 62b sowie zwei nach innen gewandte Ausstülpungen 64b vorgesehen. Im aufgesetzten Zustand, der beispielsweise in Fig. 2A zu ersehen und in Fig. 2D verdeutlicht ist, befinden sich die Ausstülpungen 64b in den Ausnehmungen 64a und das Außengewinde 62a ist im Eingriff mit dem Innengewinde 62b. Zum Lösen der Kappe ist es erforderlich, zwei mit Griffrippen versehene Griffflächen 53 an der Kappe 52 aufeinander zu zu drücken. In der in Fig. 2D durch Pfeile verdeutlichten Weise führt dies zu einem Ausrücken der Ausstülpungen 64b aus den Ausnehmungen 64a. Somit kann nach einer derartigen Kraftbeaufschlagung der Kappe 52 diese abgeschraubt werden. Um dies zu erleichtern, sind auch am Befestigungsabschnitt um 90° zu den Griffflächen 53 im aufgesetzten Zustand der Kappe 52 Griffflächen 66 vorgesehen. Diese Anordnung der Griffflächen 66 im Befestigungsabschnitt bietet gegenüber anderweitigen Griffflächen an der Basis oder dem Applikator den Vorteil, dass eine Drehbeweglichkeit des Befestigungsabschnitts 58 gegenüber dem Applikator 18 einem Lösen der Kappe nicht entgegensteht. Dennoch wird es als vorteilhaft angesehen, wenn die im Bereich der Kopplungsnut 61a und des Kopplungsstegs 61b geschaffene Verbindung einer Verdrehung des Befestigungsabschnitts 58 gegenüber dem Applikator 18 entgegenwirkt. Dies kann durch eine nur mit hoher Kraft zu überwindende kraftschlüssige Verbindung oder auch durch Formschluss geschehen, sofern die Kopplungsnut 61a einen solchen Formschluss in rotativer Hinsicht gestattet.

Wie sich Fig. 2C entnehmen lässt, liegt die Innenseite der Stirnfläche der Kappe 52 im aufgesetzten Zustand der Kappe unmittelbar an der Austragöffnung an, so dass keine Flüssigkeit entweichen kann. Hierdurch ist gewährleistet, dass keine Flüssigkeit in den Zwischenbereich zwischen Kappe 52 und Austragöffnung 22 gelangt. Die Betätigung des Spenders, also die Verlagerung des Applikators 18 gegenüber der Basis 16, ist daher bei aufgesetzter Kappe 52 ebenfalls nicht möglich.

Die Ausführungsform der Fig. 3A bis 3F weist eine hohe Verwandtschaft zur Ausführungsform der Fig. 2A bis 2F auf. Die Wechselwirkung zwischen dem Befestigungsabschnitt 58 und der Kappe 52 der Kappeneinheit 50 ist identisch mit der vorangegangenen Ausführungsform. Die Besonderheit bei dieser Gestaltung liegt darin, dass der Befestigungsabschnitt 58 die Fingerauflage 19 des nicht kindergesicherten Spenders nicht ersetzt, sondern in der insbesondere Fig. 3C entnehmbaren Weise umgreift. Ein sich hieraus ergebender Vorteil liegt darin, dass mit Ausnahme der Kappe 13 der nicht kindergesicherte Spender der Fig. 1A bis 1D vollständig montiert werden kann, bevor die Entscheidung getroffen wird, ob er mit einer Kindersicherung versehen werden soll. Dies erhöht nochmals die Flexibilität in der Produktionssteuerung. Auch kann so eine Kindersicherung bei Applikatoren ergänzt werden, an denen die Fingerauflage einstückig angeformt ist.

Die Ausgestaltung der Fig. 4A bis 4E weist ebenfalls eine große Verwandtschaft zur Ausgestaltung der Fig. 2A bis 2F auf. Im Falle der Ausgestaltung der Fig. 4A bis 4E ist die Innenseite des Befestigungsabschnitts 52 der Kappeneinheit 50 identisch mit der Ausführungsform der Fig. 2A bis 2F ausgebildet. Dies bedeutet, dass ebenfalls eine Befestigung des Befestigungsabschnitts 58 am Applikator über einen nach innen gewandten Kopplungssteg 61b und eine nach außen gewandte Kopplungsnut 61a erfolgt. Auch der genannte Hülsenabschnitt 68b ist vorgesehen.

Die Besonderheit bei der Ausgestaltung der Fig. 4A bis 4E liegt in der Kindersicherung selbst. Die Kappe 52 dieses Ausführungsbeispiels ist als Doppelkappe ausgestaltet und weist eine Innenkappe 54a sowie eine Außenkappe 54b auf. Diese beiden Teilkappen sind bestimmungsgemäß nicht voneinander trennbar, jedoch grundsätzlich gegeneinander drehbeweglich. Durch ineinandergreifende Konturen 56 ist es jedoch möglich, durch Kraftbeaufschlagung der Außenkappe 54b nach unten die Außenkappe 54b formschlüssig mit der Innenkappe 54a zu koppeln, so dass anschließend eine gemeinsame Drehbewegung möglich wird. An der Innenseite der Innenkappe 54a ist wiederum ein Innengewinde 62b vorgesehen, welches mit einem Außengewinde 62a am Befestigungsabschnitt 52 zusammenwirkt. Auf eine zusätzliche Drehsicherung entsprechend der der Fig. 2A bis 2F kann jedoch verzichtet werden. Die Schwierigkeit für ein Kind liegt darin, die Koppelbarkeit der Außenkappe 54b und der Innenkappe 54a zu durchschauen. Es wird demnach verhindert, dass das Kind überhaupt in der Lage ist, im Bereich des Gewindes 62 ein Drehmoment einzukoppeln. Eine zusätzliche Verformung der Kappe zum Zwecke der Trennung einer Drehsicherung ist daher nicht erforderlich.

## Patentansprüche

1. Austragkopf (12) für einen Spender (10) zum Austrag einer Flüssigkeit, insbesondere zum Austrag einer pharmazeutischen oder kosmetischen Flüssigkeit, mit den Merkmalen:
a. der Austragkopf (12) umfasst eine Basis (16) und einen beweglich an der Basis (16) angebrachten Applikator (18), und
b. die Basis (16) ist zur Montage an einem Flüssigkeitsspeicher (20) oder zur festen Integration an einen Flüssigkeitsspeicher ausgebildet, und
c. der Applikator (18) umfasst eine Austragöffnung (22), durch die Flüssigkeit in eine Umgebung abgegeben werden kann, und
d. der Austragkopf (12) umfasst eine Pumpeinrichtung (24) oder eine Ventileinrichtung, die in einem Flüssigkeitskanal (26) angeordnet ist, die den Flüssigkeitsspeicher (20) mit der Austragöffnung (22) verbindet, wobei die Pumpeinrichtung (24) durch Verlagerung des Applikators gegenüber der Basis (16) betätigbar ist oder wobei die Ventileinrichtung durch Verlagerung des Applikators gegenüber der Basis geöffnet und geschlossen werden kann,
e. der Austragkopf (12) umfasst eine kindergesicherte Kappeneinheit (50), und
f. die Kappeneinheit (50) umfasst eine Kappe (52) zur Überdeckung der Austragöffnung (22), **gekennzeichnet durch** die Merkmale:
g. die Kappeneinheit (50) umfasst einen separaten Befestigungsabschnitt (58), der über eine Befestigungseinrichtung (60) einerseits fest am Applikator (18) angebracht ist und der andererseits zur lösbaren Befestigung der Kappe (52) ausgebildet ist, und
h. der Befestigungsabschnitt (58) und die Kappe (52) der Kappeneinheit (50) sind mit einer Gewindeeinrichtung (62) oder einer Bajonetteinrichtung zum Befestigen der Kappe (52) am Befestigungsabschnitt (58) versehen.

2. Austragkopf (12) nach Anspruch 1 mit dem Merkmal:
a. der Befestigungsabschnitt (58) der Kappeneinheit (50) ist als zumindest abschnittsweise hülsenförmiger und beidseitig offener Körper gestaltet, der den Applikator (18) umgibt.

3. Austragkopf (12) nach einem der vorstehenden Ansprüche mit den Merkmalen:
a. der Befestigungsabschnitt (58) und die Kappe (52) der Kappeneinheit (50) verfügen über eine Drehsicherungseinrichtung (64), durch die die Kappe (52) und der Befestigungsabschnitt (58) in einer definierten Drehstellung formschlüssig gegen Verdrehen gesichert werden können, und
b. der Befestigungsabschnitt (58) oder die Kappe (52) sind abschnittsweise derart verformbar, dass hierdurch die durch die Drehsicherungseinrichtung (64) herstellbare Drehsicherung lösbar ist.

4. Austragkopf (12) nach einem der vorstehenden Ansprüche mit den Merkmalen:
a. die Kappe (52) ist als Doppelkappe mit einer Innenkappe (54a) und einer Außenkappe (54b) gestaltet, und
b. die Innenkappe (54a) und die Außenkappe (54b) verfügen über zusammenwirkende Kopplungsmittel (56), durch die durch Kraftbeaufschlagung der Außenkappe (54b) eine temporäre Drehsicherung zwischen der Außenkappe (54b) und der Innenkappe (54a) herstellbar ist.

5. Austragkopf (12) nach einem der vorstehenden Ansprüche mit mindestens einem der Merkmale:
a. die Befestigungseinrichtung (60) umfasst eine Rastverbindungseinrichtung (61), oder
b. die Befestigungseinrichtung (60) ist als drehfeste Befestigungseinrichtung (60) ausgebildet, durch die der Befestigungsabschnitt (58) einerseits und der Applikator (18) andererseits drehfest miteinander verbunden sind.

6. Austragkopf (12) nach einem der vorstehenden Ansprüche mit den Merkmalen:
a. der Applikator (18) des Austragkopfes (12) weist eine Fingerauflage (19) auf, und
b. der Befestigungsabschnitt (58) ist durch außenseitiges Umgreifen der Fingerauflage (19) am Applikator befestigt.

7. Austragkopf (12) nach einem der vorstehenden Ansprüche mit den Merkmalen:
a. die Befestigungseinrichtung (60) ist zur Kopplung des Befestigungsabschnitts (58) am Applikator (18) ausgebildet, und
b. die Befestigungseinrichtung (60) umfasst eine radial nach außen offene Kopplungsnut (61a), in den ein innenseitig am Befestigungsabschnitt (58) vorgesehener Kopplungssteg (61b) eingreift.

8. Austragkopf (12) nach einem der vorstehenden Ansprüche mit mindestens einem der folgenden Merkmale:
a. der Befestigungsabschnitt (58) der Kappeneinheit (50) verfügt über eine Grifffläche (66), die zur Erhöhung der Griffigkeit mit Griffrippen oder anderweitigen Grifferhebungen versehen ist, oder
b. der Befestigungsabschnitt (58) der Kappeneinheit (50) umfasst eine Fingerauflage (68), die einen Außenrand aufweist, dessen Formgebung von der Kreisform abweicht.

9. Austragkopf (12) nach einem der vorstehenden Ansprüche mit mindestens einem der folgenden Merkmale:
a. der Applikator (18) ist als Nasalapplikator ausgebildet und verfügt über eine Nasenolive (18a), an deren distalem Ende die Austragöffnung (22) vorgesehen ist, oder
b. der Applikator (18) ist als Applikator zur Tropfenabgabe ausgebildet und verfügt über eine die Austragöffnung umgebende Tropfenbildungsfläche, an der ausgetragenen Flüssigkeit anhaftet, bis sie sich in Tropfenform hiervor löst, oder
c. der Applikator (18) weist eine sich in Richtung auf die Austragöffnung (22) zu verjüngende außenseitige Formgebung auf, und der Befestigungsabschnitt (58) weist eine zur dieser Formgebung des Applikators (18) korrespondierende innenseitige sich verjüngende Formgebung auf, oder
d. an der Fingerauflage (68) ist ein umlaufender Hülsenabschnitt (68b) vorgesehen, der die Basis (16) umgibt, oder
e. die Kappe (52) verfügt an ihrer Innenseite über eine Dichtfläche, mittels derer im aufgesetzten Zustand die Austragöffnung (22) unmittelbar verschlossen wird, oder
f. die Kappe (52) weist einen Blockierabschnitt auf, der zusammen mit einem weiteren Blockierabschnitt an der Basis im aufgesetzten Zustand der Kappe ein Niederdrücken des Applikators gegenüber der Basis verhindert.

10. Spender (10) zum Austrag einer Flüssigkeit, insbesondere zum Austrag einer pharmazeutischen oder kosmetischen Flüssigkeit, mit den Merkmalen:
a. der Spender (10) umfasst einen Flüssigkeitsspeicher (20), und
b. der Spender (10) umfasst einen Austragkopf (12) mit einer Austragöffnung (22), durch die Flüssigkeit in eine Umgebung abgebbar ist,
**gekennzeichnet durch** das Merkmal:
c. der Austragkopf (12) ist nach einem der Ansprüche 1 bis 9 ausgebildet.

11. Spender (10) nach Anspruch 10 mit dem Merkmal:
a. der Flüssigkeitsspeicher (20) weist ein Volumen von maximal 1000ml auf, vorzugsweise von maximal 100ml, insbesondere vorzugsweise von maximal 50ml.

## Claims

1. Discharge head (12) for a dispenser (10) for discharging a liquid, in particular for discharging a pharmaceutical or cosmetic liquid, with the features:
a. the discharge head (12) comprises a base (16) and an applicator (18) mounted movably on the base (16), and
b. the base (16) is designed to be fitted onto a liquid reservoir (20) or to be fixedly integrated on a liquid reservoir, and
c. the applicator (18) comprises a discharge opening (22) through which liquid can be released into an environment, and
d. the discharge head (12) comprises a pump mechanism (24) or a valve mechanism, which is arranged in a liquid channel (26) connecting the liquid reservoir (20) to the discharge opening (22), wherein the pump mechanism (24) can be actuated by displacement of the applicator with respect to the base (16), or wherein the valve mechanism can be opened and closed by displacement of the applicator with respect to the base,
e. the discharge head (12) comprises a childproof cap unit (50), and
f. the cap unit (50) comprises a cap (52) for covering the discharge opening (22), **characterized by** the features:
g. the cap unit (50) comprises a separate securing portion (58) which, on the one hand, is mounted securely on the applicator (18) via a securing mechanism (60) and, on the other hand, is designed to secure the cap (52) releasably, and
h. the securing portion (58) and the cap (52) of the cap unit (50) are provided with a thread mechanism (62) or a bayonet mechanism for securing the cap (52) on the securing portion (58) .

2. Discharge head (12) according to Claim 1, with the feature:
a. the securing portion (58) of the cap unit (50) is designed as a body which is sleeve-shaped at least in part and is open at both ends and surrounds the applicator (18).

3. Discharge head (12) according to one of the preceding claims, with the features:
a. the securing portion (58) and the cap (52) of the cap unit (50) have an anti-rotation mechanism (64), by means of which the cap (52) and the securing portion (58) can be made secure against rotation, by form-fit engagement, in a defined rotation position, and
b. the securing portion (58) and the cap (52) are deformable in part, in such a way that the rotation block that can be produced by the anti-rotation mechanism (64) is thus releasable.

4. Discharge head (12) according to one of the preceding claims, with the features:
a. the cap (52) is designed as a double cap with an inner cap (54a) and an outer cap (54b), and
b. the inner cap (54a) and the outer cap (54b) have interacting coupling means (56), by which a temporary rotation block between the outer cap (54b) and the inner cap (54a) can be produced by applying force to the outer cap (54b).

5. Discharge head (12) according to one of the preceding claims, with at least one of the features:
a. the securing mechanism (60) comprises a latching connection mechanism (61), or
b. the securing mechanism (60) is designed as a rotationally fixed securing mechanism (60), by which the securing portion (58), on the one hand, and the applicator (18), on the other hand, are connected to each other for conjoint rotation.

6. Discharge head (12) according to one of the preceding claims, with the features:
a. the applicator (18) of the discharge head (12) has a finger support (19), and
b. the securing portion (58) is secured on the applicator by engagement around the outside of the finger support (19).

7. Discharge head (12) according to one of the preceding claims, with the features:
a. the securing mechanism (60) is designed for coupling the securing portion (58) to the applicator (18), and
b. the securing mechanism (60) comprises a radially outwardly open coupling groove (61a), in which a coupling web (61b) provided internally on the securing portion (58) engages.

8. Discharge head (12) according to one of the preceding claims, with at least one of the following features:
a. the securing portion (58) of the cap unit (50) has a gripping surface (66) which, in order to increase the grippability, is provided with gripping ribs or other kinds of gripping elevations, or
b. the securing portion (58) of the cap unit (50) comprises a finger support (68), which has an outer edge whose shape deviates from the shape of a circle.

9. Discharge head (12) according to one of the preceding claims, with at least one of the following features:
a. the applicator (18) is designed as a nasal applicator and has a nosepiece (18a), at the distal end of which the discharge opening (22) is provided, or
b. the applicator (18) is designed as an applicator for releasing drops and has a drop formation surface which surrounds the discharge opening and on which emerging liquid attaches itself until it detaches therefrom in the form of a drop, or
c. the applicator (18) has an external shape that narrows in the direction of the discharge opening (22), and the securing portion (58) has a shape that narrows on the inside in a manner corresponding to this shape of the applicator (18), or
d. a circumferential sleeve portion (68b) is provided on the finger support (68) and surrounds the base (16), or
e. the cap (52) has, on its inside, a sealing surface which, when the cap (52) is fitted, directly closes the discharge opening (22), or
f. the cap (52) has a blocking portion which, together with a further blocking portion on the base, prevents the applicator from being pressed down with respect to the base when the cap is fitted.

10. Dispenser (10) for discharging a liquid, in particular for discharging a pharmaceutical or cosmetic liquid, with the features:
a. the dispenser (10) comprises a liquid reservoir (20), and
b. the dispenser (10) comprises a discharge head (12) with a discharge opening (22) through which liquid can be released into an environment,
**characterized by** the feature:
c. the discharge head (12) is designed according to one of Claims 1 to 9.

11. Dispenser (10) according to Claim 10, with the feature:
a. the liquid reservoir (20) has a maximum volume of 1000 ml, preferably a maximum volume of 100 ml, particularly preferably a maximum volume of 50 ml.

## Revendications

1. Tête de distribution (12) pour un distributeur (10) pour la distribution d'un liquide, en particulier pour la distribution d'un liquide pharmaceutique ou cosmétique, comprenant les caractéristiques suivantes :
a. la tête de distribution (12) comprend une base (16) et un applicateur (18) monté de manière déplaçable sur la base (16), et
b. la base (16) est réalisée pour le montage sur un réservoir de liquide (20) ou pour l'intégration fixe sur un réservoir de liquide, et
c. l'applicateur (18) comprend une ouverture de distribution (22) à travers laquelle le liquide peut être distribué dans un environnement, et
d. la tête de distribution (12) comprend un dispositif de pompe (24) ou un dispositif de valve qui est disposé dans un conduit de liquide (26) qui relie le réservoir de liquide (20) à l'ouverture de distribution (22), le dispositif de pompe (24) pouvant être actionné par le déplacement de l'applicateur par rapport à la base (16), ou le dispositif de valve pouvant être ouvert et fermé par déplacement de l'applicateur par rapport à la base,
e. la tête de distribution (12) comprend une unité de capuchon (50) à sécurité enfants, et
f. l'unité de capuchon (50) comprend un capuchon (52) destiné à recouvrir l'ouverture de distribution (22),
**caractérisée par** les caractéristiques suivantes :
g. l'unité de capuchon (50) comprend une portion de fixation séparée (58) qui est d'une part montée fixement sur l'applicateur (18) par le biais d'un dispositif de fixation (60) et qui d'autre part est réalisée pour la fixation libérable du capuchon (52), et
h. la portion de fixation (58) et le capuchon (52) de l'unité de capuchon (50) sont pourvus d'un dispositif de filetage (62) ou d'un dispositif de baïonnette pour la fixation du capuchon (52) sur la portion de fixation (58).

2. Tête de distribution (12) selon la revendication 1, comprenant la caractéristique suivante :
a. la portion de fixation (58) de l'unité de capuchon (50) est configurée sous forme de corps au moins en partie en forme de douille et ouvert des deux côtés, qui entoure l'applicateur (18).

3. Tête de distribution (12) selon l'une quelconque des revendications précédentes, comprenant les caractéristiques suivantes :
a. la portion de fixation (58) et le capuchon (52) de l'unité de capuchon (50) disposent d'un dispositif de fixation en rotation (64), par lequel le capuchon (52) et la portion de fixation (58) peuvent être fixés contre toute rotation dans une position de rotation définie par engagement par correspondance de formes, et
b. la portion de fixation (58) ou le capuchon (52) peuvent être déformés en partie de telle sorte que la fixation en rotation pouvant être établie par le dispositif de fixation en rotation (64) puisse ainsi être desserrée.

4. Tête de distribution (12) selon l'une quelconque des revendications précédentes, comprenant les caractéristiques suivantes :
a. le capuchon (52) est configuré sous forme de double capuchon avec un capuchon intérieur (54a) et un capuchon extérieur (54b), et
b. le capuchon intérieur (54a) et le capuchon extérieur (54b) disposent de moyens d'accouplement coopérants (56), par le biais desquels, par une sollicitation par force du capuchon extérieur (54b), une fixation en rotation temporaire entre le capuchon extérieur (54b) et le capuchon intérieur (54a) peut être établie.

5. Tête de distribution (12) selon l'une quelconque des revendications précédentes, comprenant au moins l'une des caractéristiques suivantes :
a. le dispositif de fixation (60) comprend un dispositif de connexion par encliquetage (61), ou
b. le dispositif de fixation (60) est réalisé sous forme de dispositif de fixation solidaire en rotation (60), par le biais duquel la portion de fixation (58) d'une part et l'applicateur (18) d'autre part sont connectés l'un à l'autre de manière solidaire en rotation.

6. Tête de distribution (12) selon l'une quelconque des revendications précédentes, comprenant les caractéristiques suivantes :
a. l'applicateur (18) de la tête de distribution (12) présente un appui pour les doigts (19), et
b. la portion de fixation (58) est fixée sur l'applicateur par engagement par l'extérieur autour de l'appui pour les doigts (19).

7. Tête de distribution (12) selon l'une quelconque des revendications précédentes, comprenant les caractéristiques suivantes :
a. le dispositif de fixation (60) est réalisé pour l'accouplement de la portion de fixation (58) à l'applicateur (18), et
b. le dispositif de fixation (60) comprend une rainure d'accouplement ouverte radialement vers l'extérieur (61a), dans laquelle s'engage une nervure d'accouplement (61b) prévue du côté intérieur sur la portion de fixation (58).

8. Tête de distribution (12) selon l'une quelconque des revendications précédentes, comprenant au moins l'une des caractéristiques suivantes :
a. la portion de fixation (58) de l'unité de capuchon (50) dispose d'une surface de préhension (66) qui est prévue pour augmenter la préhension avec des nervures de préhension ou des rehaussements de préhension réalisés d'une autre manière, ou
b. la portion de fixation (58) de l'unité de capuchon (50) comprend un appui pour les doigts (68) qui présente un bord extérieur dont la forme s'écarte d'une forme circulaire.

9. Tête de distribution (12) selon l'une quelconque des revendications précédentes, comprenant au moins l'une des caractéristiques suivantes :
a. l'applicateur (18) est réalisé sous forme d'applicateur nasal et dispose d'une olive nasale (18a) au niveau de l'extrémité distale de laquelle est prévue l'ouverture de distribution (22), ou
b. l'applicateur (18) est un applicateur réalisé pour distribuer des gouttes et dispose d'une surface de formation de gouttes entourant l'ouverture de distribution, au niveau de laquelle le liquide distribué adhère jusqu'à ce qu'il s'en détache sous forme de goutte, ou
c. l'applicateur (18) présente une forme du côté extérieur se rétrécissant dans la direction de l'ouverture de distribution (22), et la portion de fixation (58) présente une forme du côté intérieur se rétrécissant, correspondant à cette forme de l'applicateur (18), ou
d. une portion de douille périphérique (68b) est prévue au niveau de l'appui pour les doigts (68), laquelle entoure la base (16), ou
e. le capuchon (52) dispose, sur son côté intérieur, d'une surface d'étanchéité au moyen de laquelle, dans l'état posé, l'ouverture de distribution (22) est directement fermée, ou
f. le capuchon (52) présente une portion de blocage qui, conjointement avec une autre portion de blocage au niveau de la base, dans l'état posé du capuchon, empêche un enfoncement de l'applicateur par rapport à la base.

10. Distributeur (10) pour distribuer un liquide, en particulier pour distribuer un liquide pharmaceutique ou cosmétique, comprenant les caractéristiques suivantes :
a. le distributeur (10) comprend un réservoir de liquide (20), et
b. le distributeur (10) comprend une tête de distribution (12) avec une ouverture de distribution (22) à travers laquelle le liquide peut être distribué dans un environnement, **caractérisé par** les caractéristiques suivantes :
c. la tête de distribution (12) est réalisée selon l'une quelconque des revendications 1 à 9.

11. Distributeur (10) selon la revendication 10, comprenant la caractéristique suivante :
a. le réservoir de liquide (20) présente un volume maximal de 1000 ml, de préférence de 100 ml, en particulier de préférence de 50 ml.
